(19) Europäisches Patentamt
European Patent Office
Office européen des brevets

(11) Veröffentlichungsnummer : **0 039 863**
**B1**

(12) # EUROPÄISCHE PATENTSCHRIFT

(45) Veröffentlichungstag der Patentschrift : **28.12.83**

(21) Anmeldenummer : **81103338.0**

(22) Anmeldetag : **04.05.81**

(51) Int. Cl.$^3$ : **C 07 D211/90**, A 61 K 31/44, C 07 D401/04, C 07 D405/04, C 07 D409/04, C 07 D413/04, C 07 D417/04

(54) **1,4-Dihydropyridine mit unterschiedlichen Substituenten in 2- und 6-Position, Verfahren zu ihrer Herstellung und ihre Verwendung in Arzneimitteln.**

(30) Priorität : **13.05.80 DE 3018259**

(43) Veröffentlichungstag der Anmeldung : **18.11.81 Patentblatt 81/46**

(45) Bekanntmachung des Hinweises auf die Patenterteilung : **28.12.83 Patentblatt 83/52**

(84) Benannte Vertragsstaaten : **AT BE CH DE FR GB IT LI LU NL SE**

(56) Entgegenhaltungen :
**EP-A- 0 031 801**
**DE-A- 1 792 764**
**DE-A- 2 302 866**
**DE-A- 2 756 226**
***Die Akte enthält technische Angaben, die nach dem Eingang der Anmeldung eingereicht wurden und die nicht in dieser Patentschrift enthalten sind.***

(73) Patentinhaber : **BAYER AG**
**Zentralbereich Patente, Marken und Lizenzen**
**D-5090 Leverkusen 1, Bayerwerk (DE)**

(72) Erfinder : **Wehinger, Egbert, Dr.**
**Donnenbergerstrasse 90**
**D-5620 Velbert 15 (DE)**
Erfinder : **Meyer, Horst, Dr.**
**Theodor-Heuss-Strasse 110**
**D-5600 Wuppertal 1 (DE)**
Erfinder : **Bossert, Friedrich, Dr.**
**Claudiusweg 7**
**D-5600 Wuppertal 1 (DE)**
Erfinder : **Kazda, Stanislav, Dr.**
**Gellertweg 18**
**D-5600 Wuppertal 1 (DE)**
Erfinder : **Stoepel, Kurt, Dr.**
**In den Birken 69**
**D-5600 Wuppertal 1 (DE)**
Erfinder : **Towart, Robertson, Dr.**
**Claudiusweg 9**
**D-5600 Wuppertal 1 (DE)**
Erfinder : **Vater, Wulf, Dr.**
**Menchendahler Strasse 23**
**D-5090 Leverkusen 3 (DE)**

**EP 0 039 863 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

1,4-Dihydropyridine mit unterschiedlichen Substituenten in 2- und 6-Position, Verfahren zu ihrer Herstellung und ihre Verwendung in Arzneimitteln

Die vorliegende Erfindung betrifft neue 1,4-Dihydropyridine mit unterschiedlichen Substituenten in 2- und 6-Position, mehrere Verfahren zu ihrer Herstellung sowie ihre Verwendung in Arzneimitteln, insbesondere in kreislaufbeeinflussenden Mitteln.

Es its bereits bekannt geworden, daß man 1,4-Dihydro-2,6-dimethyl-4-phenyl-pyridin-3,5-dicarbonsäurediethylester erhält, wenn man Benzylidenacetessigsäureethylester mit β-Aminocrotonsäureethylester oder Acetessigsäureethylester und Ammoniak umsetzt (E. Knoevenagel, Ber. dtsch. chem. Ges. *31*, 743 (1898)).

Weiterhin ist bekannt, daß bestimmte 1,4-Dihydropyridine interessante pharmakologische Eigenschaften aufweisen (F. Bossert, W. Vater, Naturwissenschaften *58*, 578 (1971)).

Weiterhin sind Dihydropyridine bekannt, die in 3- und 5-Position unterschiedliche Estergruppen tragen (DE-OS 2 117 571). 1,4-Dihydropyridine mit gleichen Estergruppen in 3- und 5-Position und verschiedenen Substituenten in 2- und 6-Position sind bisher noch nicht bekannt geworden.

Aus der DE-OS 1 792 764 sind symmetrische Dihydropyridinester bekannt, die in 2- und 6-Position jeweils gleiche Substituenten tragen.

In der DE-OS 2 756 226 werden ebenfalls 1,4-Dihydropyridine beschrieben, die sich von den erfindungsgemäßen Verbindungen dadurch unterscheiden, daß sie in 2-Position einen Substituenten tragen, der mindestens 1 Sauerstoff oder Stickstoff als Heteroatom enthält während die erfindungsgemäßen Verbindungen in 2-Position einen reinen Kohlenwasserstoffrest tragen.

In der DE-OS 2 302 866 werden einige Dihydropyridine als Ausgangsprodukte bzw. als Zwischenprodukte beschrieben. Eine pharmakologische Wirkung für diese 2-, 6-unsymmetrisch substituierten Zwischenprodukte ist nicht angegeben. Insbesondere sei auf das Zwischenprodukt des Beispiels 26 aus dieser Offenlegungsschrift hingewiesen, welches durch Disclaimer vom Stoffanspruch der vorliegenden Anmeldung ausgenommen wird.

Die vorliegende Erfindung betrifft neue 1,4-Dihydropyridine mit unterschiedlichen Substituenten in 2- und 6-Position der allgemeinen Formel (I)

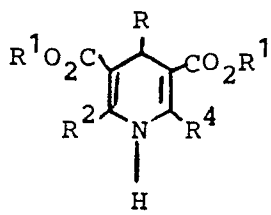

(I)

in welcher

R für einen Phenylrest steht, der gegebenenfalls 1 oder 2 gleiche oder verschiedene Substituenten aus der Gruppe Nitro, Chlor, Cyano oder Trifluormethyl enthält, ausgenommen ein gleichzeitig ortho-meta-dichlorsubstituiertes Phenyl, wenn $R_2$ Methyl und $R_4$ Alkyl mit 2-3 Kohlenstoffatomen bedeutet.

$R^1$ für einen gesättigten Kohlenwasserstoffrest mit bis zu 6 Kohlenstoffatomen steht, der gegebenenfalls durch ein Sauerstoffatom unterbrochen ist,

$R^2$ und $R^4$ immer voneinander verschieden sind und jeweils für Wasserstoff, Alkyl mit 1 bis 4 Kohlenstoffatomen oder Cyclohexylmethyl stehen, mit Ausnahme der Verbindung Diethyl-2-methyl-6-propyl-4-(3-nitrophenyl)-1,4-dihydro-3,5-carboxylat.

Es wurde gefunden, daß man die 1,4-Dihydropyridin-Derivate der Formel (I) erhält, wenn man

A) Yliden-β-ketoester der allgemeinen Formel (II)

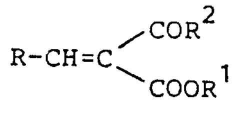

(II)

in welcher

R, $R^1$ und $R^2$ die oben angegebene Bedeutung haben, mit Enaminocarbonsäureestern der allgemeinen Formel (III)

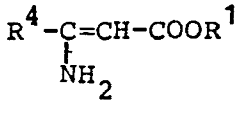

(III)

in welcher
$R^1$ und $R^4$ die oben angegebene Bedeutung haben, gegebenenfalls in Gegenwart inerter organischer Lösungsmittel umsetzt, oder
B) Yliden-β-ketoester der allgemeinen Formel (II)

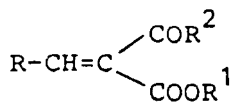
(II)

in welcher
R, $R^1$ und $R^2$ die oben angegebene Bedeutung haben, mit Aminen der allgemeinen Formel (IV) und β-Ketocarbonsäureestern der allgemeinen Formel (V)

$$-NH_3 \quad + \quad R^4-CO-CH_2-COOR^1$$

(IV) (V)

in welchen
$R^4$ und $R^1$ die oben angegebene Bedeutung haben, gegebenenfalls in Gegenwart inerter organischer Lösungsmittel umsetzt, oder
C) Yliden-β-ketoester der allgemeinen Formel (VI)

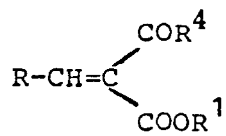
(VI)

in welcher
R, $R^1$ und $R^4$ die oben angegebene Bedeutung haben, mit Enaminocarbonsäureestern der allgemeinen Formel (VII)

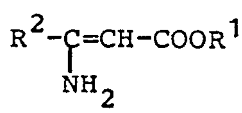
(VII)

in welcher
$R^2$ und $R^1$ die oben angegebene Bedeutung haben, gegebenenfalls in Gegenwart von inerten organischen Lösungsmitteln umsetzt, oder
D) Yliden-β-ketoester der Formel (VI)

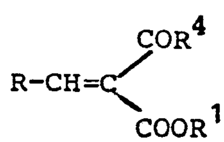
(VI)

in welcher
R, $R^1$ und $R^4$ die oben angegebene Bedeutung haben, mit Aminen der allgemeinen Formel (IV) und β-Ketocarbonsäureestern der allgemeinen Formel (VIII)

$$-NH_3 \quad + \quad R^2-CO-CH_2-COOR^1$$

(IV) (VIII)

in welchen
$R^2$ und $R^1$ die oben angegebene Bedeutung haben, gegebenenfalls in Gegenwart inerter organischer

Lösungsmittel umsetzt, oder

E) Aldehyde der allgemeinen Formel (IX)

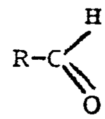

(IX)

in welcher

R die oben angegebene Bedeutung hat, mit Enaminocarbonsäureestern der allgemeinen Formel (III) und β-Ketocarbonsäureestern der allgemeinen Formel (VIII)

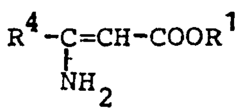

+ $R^2-CO-CH_2-COOR^1$

(III) (VIII)

in welchen

$R^4$, $R^1$ und $R^2$ die oben angegebene Bedeutung haben gegebenenfalls in Gegenwart inerter organischer Lösungsmittel umsetzt, oder

F) Aldehyde der allgemeinen Formel (IX)

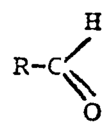

(IX)

in welcher

R die oben angegebene Bedeutung hat, mit Enaminocarbonsäureestern der allgemeinen Formel (VII) und β-Ketocarbonsäureestern der allgemeinen Formel (V)

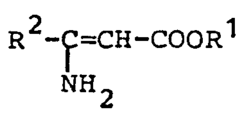

+ $R^4-CO-CH_2-COOR^1$

(VII) (V)

in welchen

$R^2$, $R^1$ und $R^4$ die oben angegebene Bedeutung haben, gegebenenfalls in Gegenwart inerter organischer Lösungsmittel zur Reaktion bringt.

Die erfindungsgemäßen 1,4-Dihydropyridin-Derivate besitzen wertvolle pharmakologische Eigenschaften. Aufgrund ihrer kreislaufbeeinflussenden Wirkung können sie als antihypertensive Mittel, als Vasodilatatoren, als Cerebraltherapeutika sowie als Coronartherapeutika Verwendung finden und sind somit als Bereicherung der Pharmazie anzusehen.

Je nach Art der verwendeten Ausgangsstoffe kann die Synthese der erfindungsgemäßen Verbindungen durch folgende Formelschemata wiedergegeben werden, wobei 1,4-Dihydro-2-ethyl-6-methyl-4-(3'-nitrophenyl)-pyridin-3,5-dicarbonsäurediethylester sowie 1,4-Dihydro-2-methyl-4-(3'-trifluormethylphenyl)-pyridin-3,5-dicarbonsäurediethylester als Beispiele gewählt seien :

A)

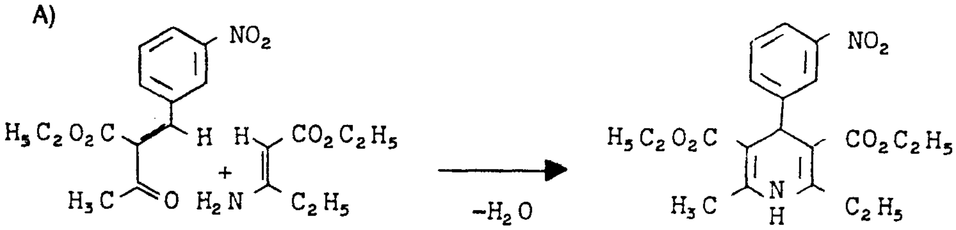

B)

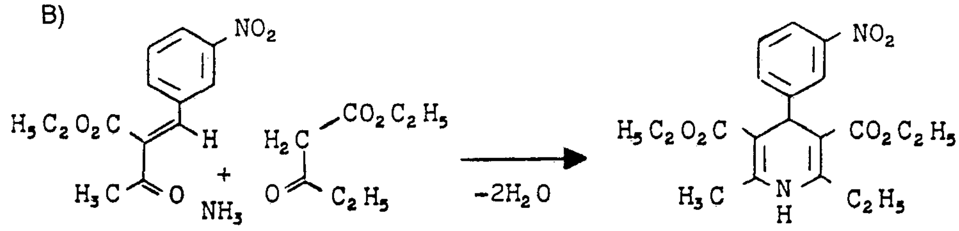


E)

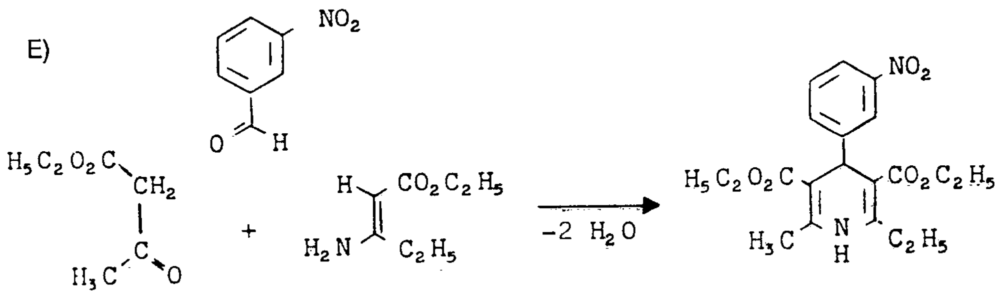


F)

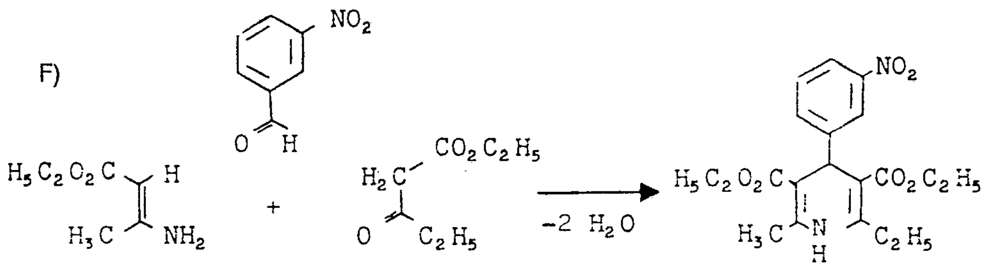


C)

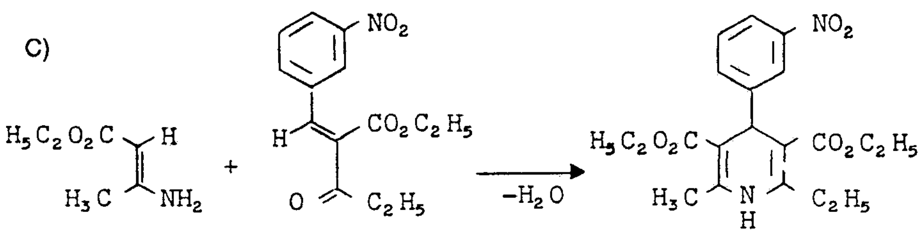


D)

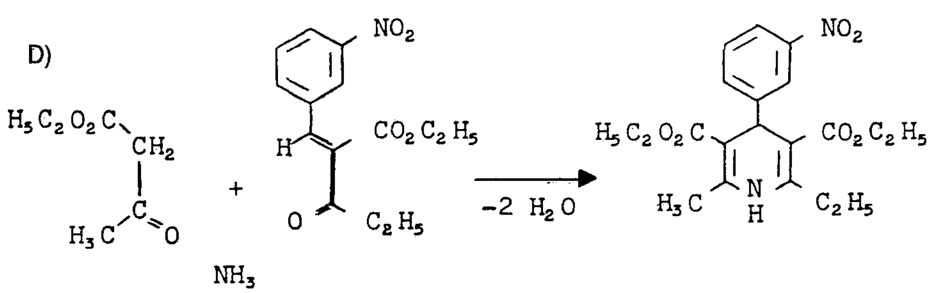


Verfahrensvariante A

Gemäß Verfahren A wird ein Yliden-β-ketoester der Formel (II)

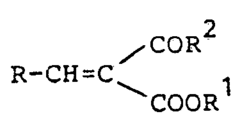

(II)

mit einem Enaminocarbonsäureester der Formel (III)

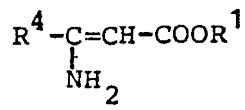

(III)

zur Reaktion gebracht.

Die als Ausgangsstoffe verwendeten Yliden-β-ketoester der Formel (II) sind literaturbekannt oder können nach literaturbekannten Methoden hergestellt werden (vgl. z. B. G. Jones « The Knoevenagel Condensation » in Org. Reactions, Vol. XV, 204 ff (1967)).

Die als Ausgangsstoffe verwendeten Enaminocarbonsäureester der Formel (III) sind literaturbekannt oder können nach literaturbekannten Methoden hergestellt werden (vgl. A. C. Cope, J. Amer. chem. Soc. *67*, 1017 (1945)).

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise zwischen 20 und 100 °C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol des Yliden-β-ketoesters der Formel (II) mit einem Mol Enaminocarbonsäureester der Formel (II) in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den gegebenenfalls erst nach Eiskühlung kristallin erhaltenen Rückstand aus einem geeigneten Lösungsmittel umkristallisiert.

Verfahrensvariante B

Gemäß Verfahren B wird ein Yliden-β-ketoester der Formel (II)

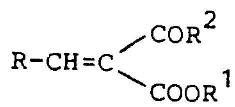

(II)

mit Ammoniak der Formel (IV) und einem β-Ketocarbonsäureester der Formel (V)

$$-NH_3 \quad + \quad R^4-CO-CH_2-COOR^1$$

(IV) (V)

zur Reaktion gebracht.

Die als Ausgangsstoffe verwendeten β-Ketocarbonsäureester der Formel (V) sind literaturbekannt und können nach literaturbekannten Methoden hergestellt werden (z. B. D. Borrmann, « Umsetzung von Diketen mit Alkoholen, Phenolen und Mercaptanen », in Houben-Weyl, Methoden der Organischen Chemie, Vol. VII/4, 230 ff (1968) ; Y. Oikawa, K. Sugano und O. Yonemitsu, J. Org. Chem. *43*, 2087 (1978)).

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formeln (II), (IV) und (V) jeweils in molaren Mengen eingesetzt. Das verwendete Amin wird zweckmäßigerweise im Überschuß von 1 bis 2 Mol zugegeben. Die erfindungsgemäßen Verbindungen können leicht durch Umkristallisation aus einem geeigneten Lösungsmittel gereinigt werden.

Verfahrensvariante C

Gemäß Verfahren C wird ein Yliden-β-ketoester der Formel (VI)

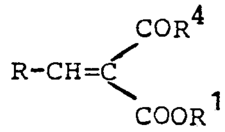

(VI)

mit einem Enaminocarbonsäureester der Formel (VII)

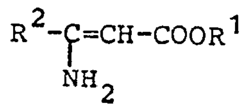

(VII)

zur Reaktion gebracht.

Die als Ausgangsstoffe verwendeten Yliden-β-ketoester der Formel (VI) sind literaturbekannt oder können nach literaturbekannten Methoden hergestellt werden (vgl. z. B. G. Jones « The Knoevenagel Condensation » in Org. Reactions, Vol. XV, 204 ff (1967)).

Die als Ausgangsstoffe verwendeten Enaminocarbonsäureester der Formel (VII) sind literaturbekannt oder können nach literaturbekannten Methoden hergestellt werden (vgl. A. C. Cope, J. Amer. Chem. Soc. *67*, 1017 (1945)).

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise zwischen 20 und 100 °C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol des Yliden-β-ketoesters der Formel (VI) mit einem Mol Enaminocarbonsäureester der Formel (VII) in einem geeigneten Lösungsmittel zur Reaktion gebracht.

Verfahrensvariante D

Gemäß Verfahren D wird ein Yliden-β-ketoester der Formel (VI)

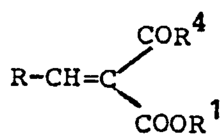

(VI)

mit Ammoniak der Formel (IV) und einem β-Ketocarbonsäureester der Formel (VIII)

$$-NH_3 \qquad + \qquad R^4-CO-CH_2-COOR^1$$

(IV) (VIII)

zur Reaktion gebracht.

Die als Ausgangsstoffe verwendeten β-Ketocarbonsäureester der Formel (VIII) sind literaturbekannt oder können nach literaturbekannten Methoden hergestellt werden (z. B. D. Borrmann, « Umsetzung von Diketen mit Alkoholen, Phenolen und Mercaptanen », in Houben-Weyl, Methoden der Organischen Chemie, Vol. VII/4, 230 ff (1968) ; Y. Oikawa, K. Sugano und O. Yonemitsu, J. Org. Chem. *43*, 2087 (1978)).

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel (VI), (IV) und (VIII) jeweils in molaren Mengen eingesetzt. Das verwendete Amin wird zweckmäßigerweise im Überschuß von 1 bis 2 Mol zugegeben.

Verfahrensvariante E

Gemäß Verfahren E wird ein Aldehyd der Formel (IX)

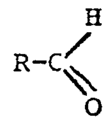

(IX)

mit einem Enaminocarbonsäureester der Formel (III)

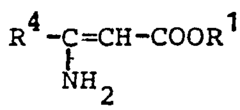

+ $R^2-CO-CH_2-COOR^1$

(III) (VIII)

und einem β-Ketocarbonsäureester der Formel (VIII) zur Reaktion gebracht.

Die als Ausgangsstoffe verwendeten Aldehyde der Formel (IX) sind literaturbekannt oder können nach literaturbekannten Methoden hergestellt werden (vgl. z. B. E. Mosettig, Org. Reactions VIII, 218 ff (1954)).

Die erfindungsgemäß verwendbaren Enaminocarbonsäureester der Formel (III) sind bereits unter Verfahrensvariante A, die erfindungsgemäß eingesetzten β-Ketocarbonsäureester der Formel (VIII) unter Verfahrensvariante D angegeben.

Als Verdünnungsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel (IX), (III) und (VIII) jeweils in molaren Mengen eingesetzt.

Verfahrensvariante F

Gemäß Verfahren F wird ein Aldehyd der Formel (IX)

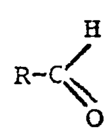

(IX)

mit einem Enaminocarbonsäureester der Formel (VII)

$$R^2-\underset{\displaystyle NH_2}{\underset{|}{C}}=CH-COOR^1 \quad + \quad R^4-CO-CH_2-COOR^1$$

(VII) (V)

und einem β-Ketocarbonsäureester der Formel (V) zur Reaktion gebracht.

Als Verdünngsmittel kommen alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150 °C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel (IX), (VII) und (V) jeweils in molaren Mengen eingesetzt.

Die vorstehenden Herstellungsverfahren sind lediglich zur Verdeutlichung der Synthese der erfindungsgemäßen Verbindungen angegeben ; jede übliche Modifikation dieser Verfahren ist in gleicher Weise für die Herstellung der erfindungsgemäßen Verbindungen der Formel I anwendbar.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Raceformen als auch die Diastereomerengemische sind Gegenstand der vorliegenden Erfindung. Die Raceformen lassen sich ebenso wie die Diastereomeren in bekannter Weise in die stereoisomer einheitlichen Bestandteile trennen (vgl. z. B. E. L. Eliel, Stereochemistry of Carbon Compounds, McGraw Hill, 1962).

Außer den unten angeführten Herstellungsbeispielen seien folgende erfindungsgemäßen Wirkstoffe genannt :

1,4-Dihydro-2-ethyl-6-methyl-4-(2′-nitrophenyl)-pyridin-3,5-dicarbonsäurediethylester,
1,4-Dihydro-2-ethyl-6-methyl-4-(2′-nitrophenyl)-pyridin-3,5-dicarbonsäuredi-n-butylester,
1,4-Dihydro-2-ethyl-6-methyl-4-(2′-nitrophenyl)-pyridin-3,5-dicarbonsäurediisobutylester,
1,4-Dihydro-2-ethyl-6-methyl-4-(2′-nitrophenyl)-pyridin-3,5-dicarbonsäure-bis-(2-methoxyethyl)ester,
1,4-Dihydro-2-ethyl-6-methyl-4-(2′-cyanophenyl)-pyridin-3,5-dicarbonsäuredimethylester,
1,4-Dihydro-2-ethyl-6-methyl-4-(2′-cyanophenyl)-pyridin-3,5-dicarbonsäuredipropylester,
1,4-Dihydro-2-ethyl-6-methyl-4-(2′-cyanophenyl)-pyridin-3,5-dicarbonsäurediisopropylester,
1,4-Dihydro-2-ethyl-6-methyl-4-(2′-trifluormethylphenyl)-pyridin-3,5-dicarbonsäuredimethylester,
1,4-Dihydro-2-ethyl-6-methyl-4-(2′-trifluormethylphenyl)-pyridin-3,5-dicarbonsäuredipropylester,
1,4-Dihydro-2-ethyl-6-methyl-4-(3′-nitrophenyl)-pyridin-3,5-dicarbonsäurediethylester,
1,4-Dihydro-2-ethyl-6-methyl-4-(3′-nitrophenyl)-pyridin-3,5-dicarbonsäuredipropylester,
1,4-Dihydro-2-ethyl-6-methyl-4-(3′-nitrophenyl)-pyridin-3,5-dicarbonsäuredicyclopentylester,
1,4-Dihydro-2-ethyl-6-methyl-4-(3′-nitrophenyl)-pyridin-3,5-dicarbonsäure-bis-(2-propoxyethyl)ester,
1,4-Dihydro-2-ethyl-6-methyl-4-(2′-chlorphenyl)-pyridin-3,5-dicarbonsäurediethylester,
1,4-Dihydro-2-propyl-6-methyl-4-(2′-nitrophenyl)-pyridin-3,5-dicarbonsäuredimethylester,
1,4-Dihydro-2-isopropyl-6-methyl-4-(2′-nitrophenyl)-pyridin-3,5-dicarbonsäuredimethylester,
1,4-Dihydro-2-propyl-6-methyl-4-(3′-nitrophenyl)-pyridin-3,5-dicarbonsäurediisopropylester,
1,4-Dihydro-2-propyl-6-methyl-4-(3′-nitrophenyl)-pyridin-3,5-dicarbonsäuredicyclopentylester,
1,4-Dihydro-2-propyl-6-methyl-4-(3′-nitrophenyl)-pyridin-3,5-dicarbonsäure-bis-(2-propoxyethyl)ester,

Die neuen Verbindungen haben ein breites und vielseitiges pharmakologisches Wirkungsspektrum. Im einzelnen konnten im Tierexperiment folgende Hauptwirkungen nachgewiesen werden :

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen nitritähnlichen herzentlastenden Effekt verstärkt.

Sie beeinflussen bzw. verändern den Herstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren. Die Verbindungen eignen sich daher besonders als Cerebraltherapeutika.

4. Die Verbindungen senken den Blutdruck von normotonen und hypertonen Tieren und können somit als antihypertensive Mittel verwendet werden.

5. Die Verbindungen haben stark muskulär-spasmolytische Wirkungen, die an der glatten Muskulatur des Magens, Darmtraktes, des Urogenitaltraktes und des Respirationssystems deutlich werden.

Die erfindungsgemäßen Verbindungen eignen sich aufgrund dieser Eigenschaften besonders zur Prophylaxe und Therapie der akuten und chronischen ischämischen Herzkrankheit im weitesten Sinne, zur Therapie des Hochdrucks sowie zur Behandlung von cerebralen und peripheren Durchblutungsstörungen.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt :

Wasser, nicht-toxische organische Lösungsmittel, wie Paraffine (z. B. Erdölfraktionen), pflanzliche Öle (z. B. Erdnuß-/Sesam-Öl), Alkohole (z. B. Ethylalkohol, Glycerin), Glykole (z. B. Propylenglykol, Polyethylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Roh-, Milch- und Traubenzucker),

Emulgiermittel (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den oben genannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von 0,001 bis 10 mg/kg, vorzugsweise 0,05 bis 5 mg/kg Körpergewicht pro Tag zur Erzielung wirksamer Ergebnisse zu verabreichen, und bei oraler Applikation beträgt die Dosierung 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1

1,4-Dihydro-2-ethyl-6-methyl-4-(2-nitrophenyl)-pyridin-3,5-dicarbonsäuredimethylester

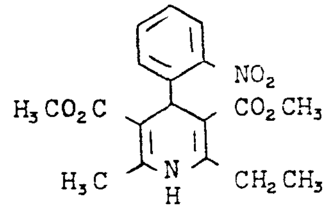

Verfahrensvariante A

Eine Lösung von 18,7 g (75 mMol) 2'-Nitrobenzylidenacetessigsäuremethylester und 9,7 g (75 mMol) β-Amino-β-ethylacrylsäuremethylester in 100 ml Methanol wurde 14 Stunden unter Stickstoff zum Sieden erhitzt. Nach dem Erkalten der Reaktionsmischung wurde das Lösungsmittel im Vakuum abdestilliert und der ölige Rückstand mit wenig Ether verrieben, wobei sich das Rohprodukt bald verfestigte. Es wurde abgesaugt und aus Methanol umkristallisiert.

Schmelzpunkt : 157 °C ; Ausbeute : 16,9 g (62 %).

Beispiel 2

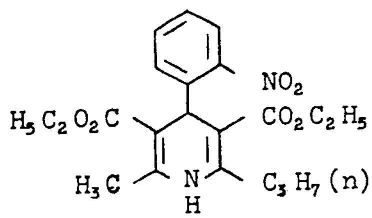

Analog Beispiel 1 wurde durch Umsetzung von 2'-Nitrobenzylidenacetessigsäureethylester mit β-Amino-β-propylacrylsäureethylester in Ethanol der 1,4-Dihydro-2-propyl-6-methyl-4-(2-nitrophenyl)-pyridin-3,5-dicarbonsäurediethylester vom Fp. : 110 °C (Ethanol) erhalten.

Ausbeute : 48 % der Theorie.

Beispiel 3

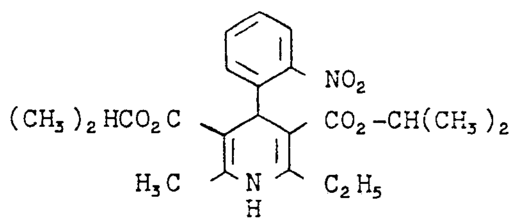

Analog Beispiel 1 wurde durch Umsetzung von 2'-Nitrobenzylidenacetessigsäureisopropylester mit β-Amino-β-ethylacrylsäureisopropylester in Isopropanol der 1,4-Dihydro-2-ethyl-6-methyl-4-(2-nitrophenyl)-pyridin-3,5-dicarbonsäurediisopropylester vom Fp : 140 °C (Ethanol) erhalten.

Ausbeute : 28 % der Theorie.

Beispiel 4

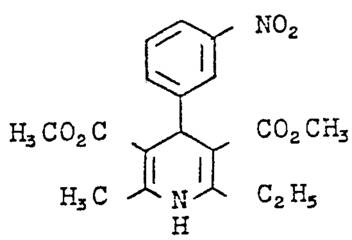

Analog Beispiel 1 wurde durch Umsetzung von 3'-Nitrobenzylidenacetessigsäuremethylester mit β-Amino-β-ethylacrylsäuremethylester in Methanol der 1,4-Dihydro-2-ethyl-6-methyl-4-(3-nitrophenyl)-pyridin-3,5-dicarbonsäuredimethylester vom Fp. : 159 °C (Methanol) erhalten.

Ausbeute : 60 % der Theorie.

Beispiel 5

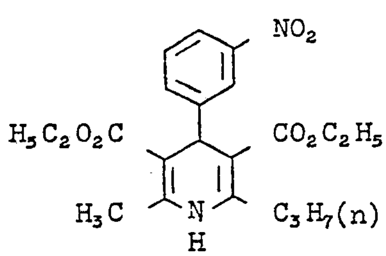

Analog Beispiel 1 wurde durch Umsetzung von 3'-Nitrobenzylidenacetessigsäureethylester mit β-Amino-β-propylacrylsäureethylester in Ethanol der 1,4-Dihydro-2-propyl-6-methyl-4-(3-nitrophenyl)-pyridin-3,5-dicarbonsäurediethylester vom Fp. : 116 °C (Ethanol) erhalten.

Ausbeute : 70 % der Theorie.

Beispiel 6

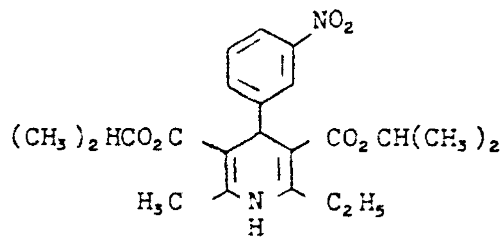

Analog Beispiel 1 wurde durch Umsetzung von 3'-Nitrobenzylidenacetessigsäureisopropylester mit β-Amino-β-ethylacrylsäureisopropylester in Isopropanol der 1,4-Dihydro-2-ethyl-6-methyl-4-(3-nitrophenyl)-pyridin-3,5-dicarbonsäurediisopropylester vom Fp. : 108 °C (Methanol) erhalten.

Ausbeute : 22 % der Theorie.

Beispiel 7

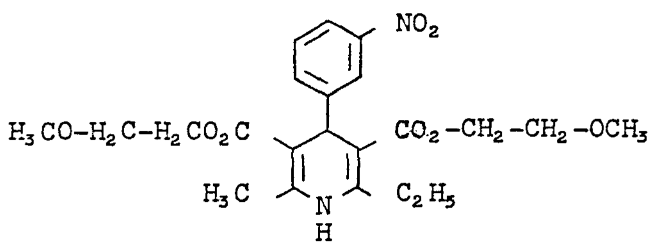

Analog Beispiel 1 wurde durch Umsetzung von 3'-Nitrobenzylidenacetessigsäure-(2-methoxyethyl)-ester mit β-Amino-β-ethylacrylsäure-(2-methoxyethyl)-ester in Ethanol der 1,4-Dihydro-2-ethyl-6-methyl-4-(3-nitrophenyl)-pyridin-3,5-dicarbonsäure-bis-(2-methoxyethyl)-ester vom Fp. : 86 °C (Methanol) erhalten.

Ausbeute : 55 % der Theorie.

Beispiel 8

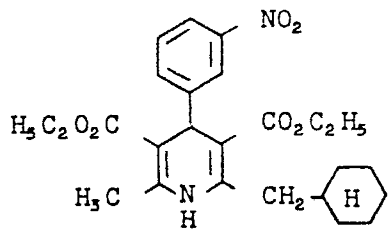

Analog Beispiel 1 wurde durch Umsetzung von 3'-Nitrobenzylidenacetessigsäureethylester mit β-Amino-β-cyclohexylmethylacrylsäureethylester in Ethanol der 1,4-Dihydro-2-cyclohexylmethyl-6-methyl-4-(3-nitrophenyl)-pyridin-3,5-dicarbonsäure-diethylester vom Fp. : 153 °C (Ethanol) erhalten.

Ausbeute : 69 % der Theorie.

Beispiel 9

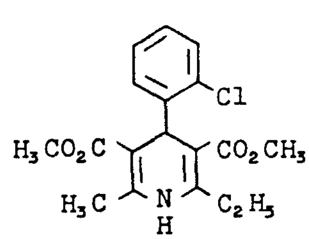

Analog Beispiel 1 wurde durch Umsetzung von 2'-Chlorbenzylidenacetessigsäuremethylester mit β-Amino-β-ethylacrylsäuremethylester in Methanol der 1,4-Dihydro-2-ethyl-6-methyl-4-(2-chlorphenyl)-pyridin-3,5-dicarbonsäuredimethylester vom Fp. : 147 °C (Methanol) erhalten.
Ausbeute : 36 % der Theorie.

Beispiel 10

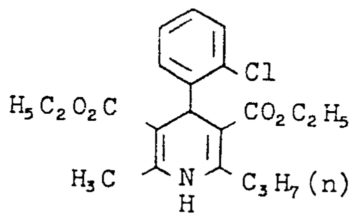

Analog Beispiel 1 wurde durch Umsetzung von 2'-Chlorbenzylidenacetessigsäureethylester mit β-Amino-β-propylacrylsäureethylester in Ethanol der 1,4-Dihydro-2-propyl-6-methyl-4-(2-chlorphenyl)-pyridin-3,5-dicarbonsäurediethylester vom Fp. : 100 °C (Ethanol) erhalten.
Ausbeute : 27 % der Theorie.

Beispiel 11

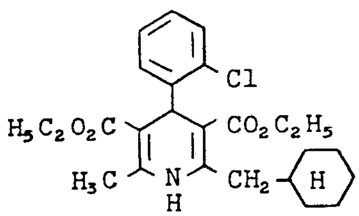

Analog Beispiel 1 wurde durch Umsetzung von 2'-Chlorbenzylidenacetessigsäureethylester mit β-Amino-β-cyclohexylmethylacrylsäureethylester in Ethanol der 1,4-Dihydro-2-cyclohexylmethyl-6-methyl-4-(2-chlorphenyl)-pyridin-3,5-dicarbonsäurediethylester vom Fp. : 117 °C (Ethanol) erhalten.
Ausbeute : 29 % der Theorie.

Beispiel 12

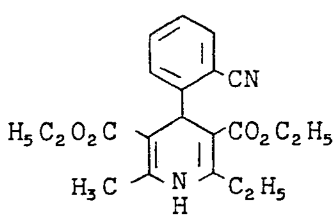

Analog Beispiel 1 wurde durch Umsetzung von 2'-Cyanobenzylidenacetessigsäureethylester mit β-Amino-β-ethylacrylsäureethylester in Ethanol der 1,4-Dihydro-2-ethyl-6-methyl-4-(2-cyanophenyl)-pyridin-3,5-dicarbonsäurediethylester vom Fp. : 123 °C (Ethanol) erhalten.
Ausbeute : 47 % der Theorie.

Beispiel 13

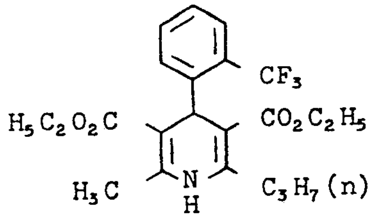

Analog Beispiel 1 wurde durch Umsetzung von 2'-Trifluormethylbenzylidenacetessigsäureethylester mit β-Amino-β-propylacrylsäureethylester in Ethanol der 1,4-Dihydro-2-propyl-6-methyl-4-(2-trifluormethylphenyl)-pyridin-3,5-dicarbonsäurediethylester vom Fp. : 95 °C (Isopropanol) erhalten.
Ausbeute : 48 % der Theorie.

Beispiel 14

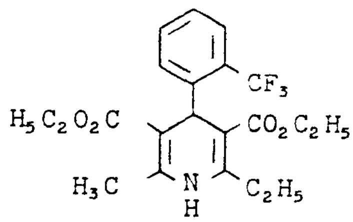

Analog Beispiel 1 wurde durch Umsetzung von 2'-Trifluormethylbenzylidenacetessigsäureethylester mit β-Amino-β-ethylacrylsäureethylester in Ethanol der 1,4-Dihydro-2-ethyl-6-methyl-4-(2-trifluormethylphenyl)-pyridin-3,5-dicarbonsäurediethylester vom Fp. : 81 °C (Isopropanol) erhalten.
Ausbeute : 62 % der Theorie.

Beispiel 15

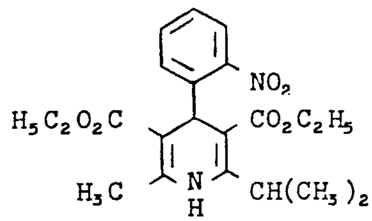

Analog Beispiel 1 wurde durch Umsetzung von 2'-Nitrobenzylidenacetessigsäureethylester mit β-Amino-β-isopropylacrylsäureethylester in Ethanol der 1,4-Dihydro-2-isopropyl-6-methyl-4-(2-nitrophenyl)-pyridin-3,5-dicarbonsäurediethylester vom Fp. : 117 °C (Ethanol) erhalten.
Ausbeute : 28 % der Theorie.

Beispiel 16

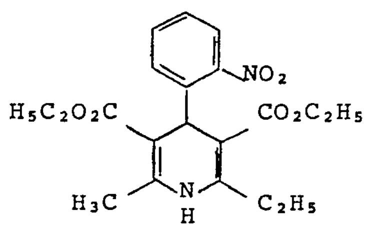

Analog Beispiel 1 wurde durch Umsetzung von 2'-Nitrobenzylidenacetessigsäureethylester mit β-Amino-β-ethylacrylsäureethylester in Ethanol der 1,4-Dihydro-2-ethyl-6-methyl-4-(2-nitrophenyl)-pyridin-3,5-dicarbonsäurediethylester vom Fp. 124 °C (Ethanol) erhalten.
Ausbeute : 35 % der Theorie.

Beispiel 17

1,4-Dihydro-2-methyl-4-(2-trifluormethylphenyl)-pyridin-3,5-dicarbonsäurediethylester

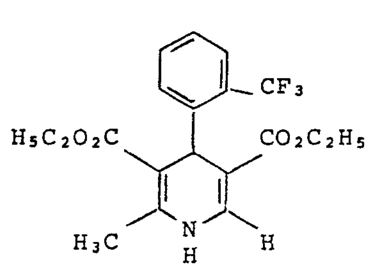

Analog Beispiel 1 wurden folgende weitere erfindungsgemäße Verbindungen (I) hergestellt :

| Bei-spiel-Nr. | R | $R^1$ | $R^2$ | $R^4$ | Summenformel | Ausbeute | Fp [°C] |
|---|---|---|---|---|---|---|---|
| 18 | 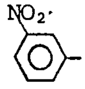 | $C_2H_5$ | $CH_3$ | H | $C_{18}H_{20}N_2O_6$ | 29% | 128-30 |
| 19 | 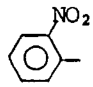 | $C_2H_5$ | $CH_3$ | H | $C_{18}H_{20}N_2O_6$ | 17% | 156-8 |
| 20 | 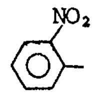 | $CH_3$ | $CH_3$ | H | $C_{16}H_{16}N_2O_6$ | 11% | 248-50 |

Analog Beispiel 20 wurden folgende weitere erfindungsgemäße Verbindungen (I) hergestellt :

| Bei-spiel-Nr. | R | $R^1$ | $R^2$ | $R^4$ | Summenformel | Ausbeute | Fp [°C] |
|---|---|---|---|---|---|---|---|
| 21 | 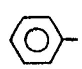 | $C_2H_5$ | $CH_3$ | H | $C_{18}H_{21}NO_4$ | 37% | 128-9 |
| 22 | 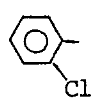 | $C_2H_5$ | $CH_3$ | H | $C_{18}H_{20}ClNO_4$ | 35% | 151 |
| 23 | 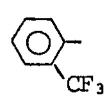 | $CH_3$ | $CH_3$ | H | $C_{17}H_{16}F_3NO_4$ | 70% | 161-3 |

**Ansprüche**

1. Verbindung der allgemeinen Formel I

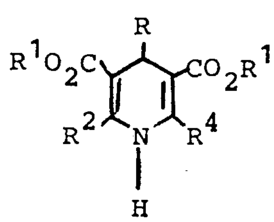

(I)

in welcher

R für einen Phenylrest steht, der gegebenenfalls 1 oder 2 gleiche oder verschiedene Substituenten aus der Gruppe Nitro, Chlor, Cyano, oder Trifluormethyl enthält, ausgenommen ein gleichzeitig ortho-meta-dichlorsubstituiertes Phenyl, wenn $R_2$ Methyl und $R_4$ Alkyl mit 2-3 Kohlenstoffatomen bedeutet

$R^1$ für einen gesättigten Kohlenwasserstoffrest mit bis zu 6 Kohlenstoffatomen steht, der gegebenenfalls durch ein Sauerstoffatom unterbrochen ist,

$R^2$ und $R^4$ immer voneinander verschieden sind und jeweils für Wasserstoff, Alkyl mit 1 bis 4 Kohlenstoffatomen oder Cyclohexylmethyl stehen, mit Ausnahme der Verbindung Diethyl-2-methyl-6-propyl-4-(3-nitrophenyl)-1,4-dihydro-3,5-carboxylat.

2. 1,4-Dihydro-2-ethyl-6-methyl-4-(2-cyanophenyl)-pyridin-3,5-dicarbonsäure-diethylester.

3. Verbindungen der allgemeinen Formel I gemäß Anspruch 1, in welcher

R, $R_1$ und $R_2$ die in Anspruch 1 angegebene Bedeutung haben, einschließlich Diethyl-2-methyl-6-propyl-4-(3-nitrophenyl)-1,4-dihydropyridin-3,5-carboxylat zur Verwendung bei der Bekämpfung von Kreislauferkrankungen.

4. Verfahren zur Herstellung von Verbindungen der allgemeinen Formel I gemäß Anspruch 1,

dadurch gekennzeichnet, daß man

A) Yliden-β-ketoester der allgemeinen Formel (II)

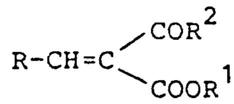

(II)

in welcher

R, $R^1$ und $R^2$ die oben angegebene Bedeutung haben, mit Enaminocarbonsäureestern der allgemeinen Formel (III)

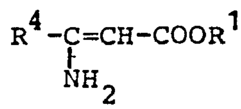

(III)

in welcher

$R^1$ und $R^4$ die oben angegebene Bedeutung haben, gegebenenfalls in Gegenwart inerter organischer Lösungsmittel umsetzt, oder

B) Yliden-β-ketoester der allgemeinen Formel (II)

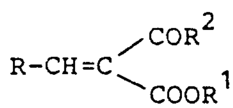

(II)

in welcher

R, $R^1$ und $R^2$ die oben angegebene Bedeutung haben, mit Aminen der allgemeinen Formel (IV) und β-Ketocarbonsäureestern der allgemeinen Formel (V)

$$-NH_3 \quad + \quad R^4-CO-CH_2-COOR^1$$

(IV) (V)

in welchen

$R^4$ und $R^1$ die oben angegebene Bedeutung haben, gegebenenfalls in Gegenwart inerter organischer Lösungsmittel umsetzt, oder

C) Yliden-β-ketoester der allgemeinen Formel (VI)

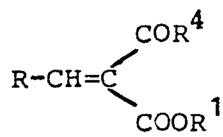

(VI)

in welcher

R, $R^1$ und $R^4$ die oben angegebene Bedeutung haben, mit Enaminocarbonsäureestern der allgemeinen Formel (VII)

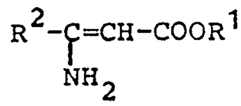

(VII)

in welcher

$R^2$ und $R^1$ die oben angegebene Bedeutung haben, gegebenenfalls in Gegenwart von inerten

organischen Lösungsmitteln umsetzt, oder

D) Yliden-β-ketoester der Formel (VI)

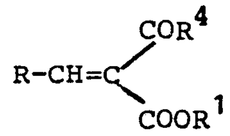

(VI)

in welcher

R, $R^1$ und $R^4$ die oben angegebene Bedeutung haben, mit Aminen der allgemeinen Formel (IV) und β-Ketocarbonsäureestern der allgemeinen Formel (VIII)

$$-NH_3 \quad + \quad R^2-CO-CH_2-COOR^1$$

(IV) (VIII)

in welchen

$R^2$ und $R^1$ die oben angegebene Bedeutung haben, gegebenenfalls in Gegenwart inerter organischer Lösungsmittel umsetzt, oder

E) Aldehyde der allgemeinen Formel (IX)

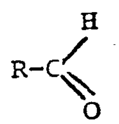

(IX)

in welcher

R die oben angegebene Bedeutung hat, mit Enaminocarbonsäureestern der allgemeinen Formel (III) und β-Ketocarbonsäureestern der allgemeinen Formel (VIII)

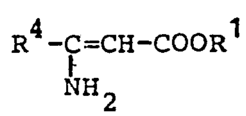

+ $R^2-CO-CH_2-COOR^1$

(III) (VIII)

in welchen

$R^4$, $R^1$ und $R^2$ die oben angegebene Bedeutung haben gegebenenfalls in Gegenwart inerter organischer Lösungsmittel umsetzt, oder

F) Aldehyde der allgemeinen Formel (IX)

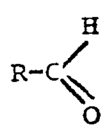

(IX)

in welcher

R die oben angegebene Bedeutung hat, mit Enaminocarbonsäureestern der allgemeinen Formel (VII) und β-Ketocarbonsäureestern der allgemeinen Formel (V)

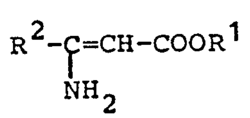

+ $R^4-CO-CH_2-COOR^1$

(VII) (V)

in welchen

$R^2$, $R^1$ und $R^4$ die oben angegebene Bedeutung haben, gegebenenfalls in Gegenwart inerter

organischer Lösungsmittel zur Reaktion bringt.

5. Arzneimittel enthaltend mindestens eine Verbindung der allgemeinen Formel (I) gemäß Anspruch 1.

6. Verfahren zur Herstellung von Arzneimitteln, dadurch gekennzeichnet, daß man mindestens eine Verbindung der allgemeinen Formel (I) gemäß Anspruch 1, gegebenenfalls unter Verwendung üblicher Hilfs- und Trägerstoffe in eine geeignete Applikationsform überführt.

**Claims**

1. Compound of the general formula I

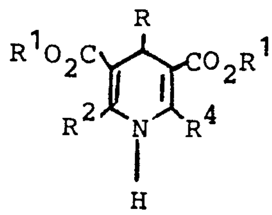

(I)

in which

R represents a phenyl radical which optionally contains 1 or 2 identical or different substituents from the group comprising nitro, chlorine, cyano or trifluoromethyl, with the exception of a phenyl radical which is at the same time ortho- and metadichlorosubstituted, when $R^2$ denotes methyl and $R^4$ denotes alkyl with 2-3 carbon atoms,

$R^1$ represents a saturated hydrocarbon radical with up to 6 carbon atoms which is optionally interrupted by an oxygen atom,

$R^2$ and $R^4$ are always different and each represents hydrogen, alkyl with 1 to 4 carbon atoms or cyclohexylmethyl, with the exception of the compound diethyl-2-methyl-6-propyl-4-(3-nitrophenyl)-1,4-dihydropyridine-3,5-carboxylate.

2. 1,4-Dihydro-2-ethyl-6-methyl-4-(2-cyanophenyl)-pyridine-3,5-dicarboxylic acid diethyl ester.

3. Compounds of the general formula I according to Claim 1, in which

R, $R^1$ and $R^2$ have the meaning indicated in Claim 1, including diethyl-2-methyl-6-propyl-4-(3-nitrophenyl)-1,4-dihydropyridine-3,5-carboxylate, for use in combating circulatory disorders.

4. Process for the preparation of compounds of the general formula (I) according to Claim 1, characterised in that

A) ylidene-β-keto-esters of the general formula (II)

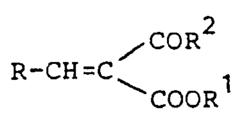

(II)

in which

R, $R^1$ and $R^2$ have the abovementioned meaning, are reacted with enaminocarboxylic acid esters of the general formula (III)

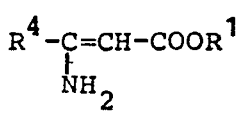

(III)

in which

$R^1$ and $R^4$ have the abovementioned meaning, optionally in the presence of inert organic solvents, or

B) ylidene-β-keto-esters of the general formula (II)

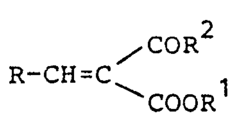

(II)

in which

R, $R^1$ and $R^2$ have the abovementioned meaning, are reacted with amines of the general formula (IV) and β-ketocarboxylic acid esters of the general formula (V)

$$-NH_3 \qquad + \qquad R^4-CO-CH_2-COOR^1$$

(IV) (V)

in which

$R^4$ and $R^1$ have the abovementioned meaning, optionally in the presence of inert organic solvents, or

C) ylidene-β-keto-esters of the general formula (VI)

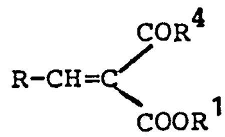

(VI)

in which

R, $R^1$ and $R^4$ have the abovementioned meaning, are reacted with enaminocarboxylic acid esters of the general formula (VII)

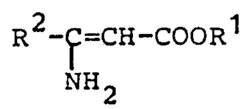

(VII)

in which,

$R^2$ and $R^1$ have the abovementioned meaning, optionally in the presence of inert organic solvents, or

D) ylidene-β-keto-esters of the formula (IV)

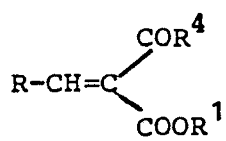

(VI)

in which

R, $R^1$ and $R^4$ have the abovementioned meaning, are reacted with amines of the general formula (IV) and β-ketocarboxylic acid esters of the general formula (VIII)

$$-NH_3 \qquad + \qquad R^2-CO-CH_2-COOR^1$$

(IV) (VIII)

in which

$R^2$ and $R^1$ have the abovementioned meaning, optionally in the presence of inert organic solvents, or

E) aldehydes of the general formula (IX)

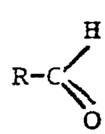

(IX)

in which

R has the abovementioned meaning, are reacted with enaminocarboxylic acid esters of the general formula (III) and β-ketocarboxylic acid esters of the general formula (VIII)

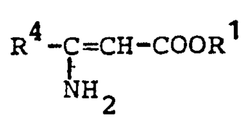

+ $R^2-CO-CH_2-COOR^1$

(III) (VIII)

in which

$R^4$, $R^1$ and $R^2$ have the abovementioned meaning, optionally in the presence of inert organic solvents, or

F) aldehydes of the general formula (IX)

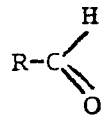

(IX)

in which

R has the abovement meaning, are reacted with enaminocarboxylic acid esters of the general formula (VII) and β-ketocarboxylic acid esters of the general formula (V)

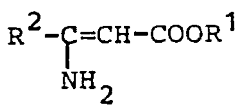 + $R^4-CO-CH_2-COOR^1$

(VII) (V)

in which

$R^2$, $R^1$ and $R^4$ have the abovementioned meaning, optionally in the presence of inert organic solvents.

5. Medicaments containing at least one compound of the general formula (I) according to Claim 1.

6. Process for the preparation of medicaments, characterised in that at least one compound of the general formula (I) according to Claim 1 is converted, optionally with the use of customary auxiliaries and excipients, into a suitable form for administration.

**Revendications**

1. Composé de formule générale I

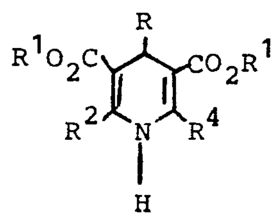

(I)

dans laquelle

R représente un radical phényle, qui contient éventuellement un ou deux substituants identiques ou différents choisis dans l'ensemble formé par un groupe nitro, chloro, cyano ou trifluorométhyle, à l'exclusion d'un groupe phényle simultanément disubstitué par du chlore en ortho et méta lorsque $R^2$ représente un groupe méthyle et $R^4$ un groupe alkyle ayant 2 ou 3 atomes de carbone,

$R^1$ représente un radical hydrocarboné saturé comportant jusqu'à 6 atomes de carbone, qui est éventuellement interrompu par un atome d'oxygène,

$R^2$ et $R^4$ sont toujours différents l'un de l'autre et représentent chacun un atome d'hydrogène, un groupe alkyle ayant 1 à 4 atomes de carbone ou un groupe cyclohexylméthyle, à l'exclusion du 2-méthyl-6-propyl-4-(3-nitrophényl)-1,4-dihydropyridine-3,5-carboxylate de diéthyle.

2. Le 1,4-dihydro-2-éthyl-6-méthyl-4-(2-cyanophényl)-pyridine-3,5-dicarboxylate de diéthyle.

3. Composés de formule générale I selon la revendication 1, dans laquelle

R, $R^1$ et $R^2$ ont le sens indiqué à la revendication 1, y compris le 2-méthyl-6-propyl-4-(3-nitrophényl)-1,4-dihydropyridine-3,5-carboxylate de diéthyle, destinés à la lutte contre les maladies de la circulation.

4. Procédé de préparation de composés de formule générale I selon la revendication 1, caractérisé en ce que :

A) on fait réagir des ylidène-β-cétoesters de formule générale (II)

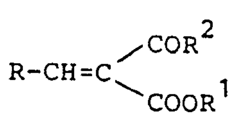

(II)

dans laquelle

R, $R^1$ et $R^2$ ont le sens indiqué ci-dessus, avec des esters d'acides énaminocarboxyliques de formule générale (III)

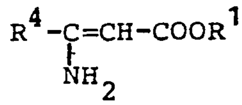

(III)

dans laquelle

$R^1$ et $R^4$ ont le sens indiqué ci-dessus, éventuellement en présence d'un solvant organique inerte, ou

B) on fait réagir des ylidène-β-cétoesters de formule générale (II)

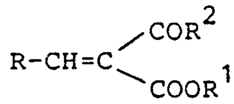

(II)

dans laquelle

R, $R^1$ et $R^2$ ont le sens indiqué ci-dessus, avec des amines de formule générale (IV) et des esters d'acides β-cétocarboxyliques de formule générale (V)

$$-NH_3 \quad + \quad R^4-CO-CH_2-COOR^1$$

(IV) (V)

dans laquelle

$R^4$ et $R^1$ ont le sens indiqué ci-dessus, éventuellement en présence d'un solvant organique inerte, ou

C) on fait réagir des ylidènes-β-cétoesters de formule générale (VI)

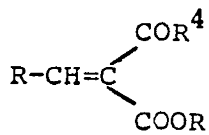

(VI)

dans laquelle

R, $R^1$ et $R^4$ ont le sens indiqué ci-dessus, avec des esters d'acides énaminocarboxyliques de formule générale

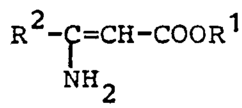

(VII)

dans laquelle

$R^2$ et $R^1$ ont le sens indiqué ci-dessus, éventuellement en présence de solvants organiques inertes, ou

D) on fait réagir des ylidènes-β-cétoesters de formule (VI)

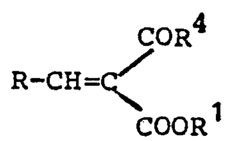

(VI)

dans laquelle

R, $R^1$ et $R^4$ ont le sens indiqué ci-dessus, avec des amines de formule générale (IV) et des esters d'acides β-cétocarboxyliques de formule générale (VIII)

$$-NH_3 \quad + \quad R^2-CO-CH_2-COOR^1$$

(IV) (VIII)

dans lesquelles

$R^2$ et $R^1$ ont le sens indiqué ci-dessus, éventuellement en présence d'un solvant organique inerte, ou

E) on fait réagir des aldéhydes de formule générale (IX)

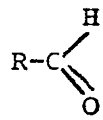

(IX)

dans laquelle

R a le sens indiqué ci-dessus, avec des esters d'acides énaminocarboxyliques de formule générale (III) et des esters d'acides β-cétocarboxyliques de formule générale (VIII)

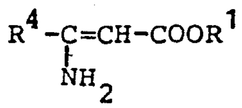 + $R^2-CO-CH_2-COOR^1$

(III) (VIII)

dans lesquelles

$R^4$, $R^1$ et $R^2$ ont le sens indiqué ci-dessus, éventuellement en présence d'un solvant organique inerte, ou

F) on fait réagir des aldéhydes de formule générale (IX)

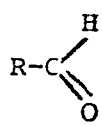

(IX)

dans laquelle

R a le sens indiqué ci-dessus, avec des esters d'acides énaminocarboxyliques de formule générale (VII) et des esters d'acides β-cétocarboxyliques de formule générale (V)

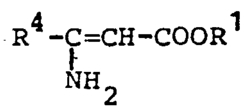 + $R^2-CO-CH_2-COOR^1$

(VII) (V)

dans lesquelles

$R^2$, $R^1$ et $R^4$ ont le sens indiqué ci-dessus, éventuellement en présence d'un solvant organique inerte.

5. Médicament contenant au moins un composé de formule générale (I) selon la revendication 1.

6. Procédé de préparation de médicaments, caractérisé en ce qu'on transforme au moins un composé de formule générale (I) selon la revendication 1, éventuellement en utilisant des adjuvants et supports usuels, en une forme convenant pour l'application.